# EUROPEAN PATENT APPLICATION

(11) **EP 1 561 808 A1**
(43) Date of publication of application: **10.08.2005**
(21) Application number: 03758895.1
(22) Date of filing: 24.10.2003
(51) Int. Cl.: C12N 1/14

(54) **PROCESS FOR PRODUCING MATSUTAKE MUSHROOM MYCELIUM**

(30) Priority: 25.10.2002 JP 2002311840
(71) Applicant: Kureha Chemical Industry Co., Ltd., Tokyo 103-8552 (JP)
(72) Inventor: KITAGOU, Hiroyuki, Iwaki-shi, Fukushima 979-0141 (JP); OOTOMO, Nobuo, Iwaki-shi, Fukushima 979-0145 (JP); NEMOTO, Yasumitu, Iwaki-shi, Fukushima 974-8232 (JP); TAKAHASHI, Eisaku, Tukuba-shi, Ibaraki 300-3261 (JP); HIWATASHI, Junji, Iwaki-shi, Fukushima 974-8212 (JP); MATSUNAGA, Kenichi, Tokorozawa-shi, Saitama 359-1142 (JP)
(74) Representative: Cohausz & Florack
(86) International application number: PCT/JP2003/013657
(87) International publication number: WO 2004/038009

(57) **Abstract**

Disclosed is a process for producing a mycelium of a Matsutake fungus, comprising the step of cultivating a mycelium on a small scale under agitation without aeration or with aeration at a low rate of less than 0.05 vvm in a liquid medium. According to the process, a large number of mycelia can be produced without a loss of physiological activities.

## Description

### TECHNICAL FIELD

The present invention relates to a process for producing mycelia of a Matsutake fungus (Matsutake mycelia). In particular, the present invention relates to a process for producing Matsutake mycelia useful as a seed culture for a large-scale production of Matsutake mycelia, and a process for producing a large number of Matsutake mycelia using the seed culture.

### BACKGROUND ART

International Publication WO02/30440 (patent reference 1) discloses that dry powder of Tricholoma matsutake mycelia is useful in promoting a recovery from stress (page 27).

Japanese Examined Patent Publication (Kokoku) No. 61-53032 (patent reference 2) discloses a process for producing Tricholoma matsutake mycelia comprising the steps of cultivating a slant culture of Tricholoma matsutake mycelia in a liquid medium containing starch under shaking for 30 days, inoculating a liquid medium (20 L) containing starch, and carrying out a further cultivation under aeration and agitation for 30 days (Example 1).

Further, Japanese Unexamined Patent Publication (Kokai) No. 11-318433 (patent reference 3) discloses a process for producing Tricholoma matsutake mycelia comprising the steps of cultivating mycelia isolated from a commercially available fruit body of Tricholoma matsutake on a plate for 4 days, carrying out a cultivation for acclimation in a liquid medium containing vegetable extracts for 4 days, and carrying out a further cultivation in a liquid medium (2 L) containing vegetable extracts using a tank for submerged cultivation under aeration and agitation for 6 days (Example 1).

However, a new process for a large-scale production in which a large number of Tricholoma matsutake mycelia can be produced without a loss of physiological activities is desired.
(patent reference 1) International Publication WO02/30440 (patent reference 2) Japanese Examined Patent Publication (Kokoku) No. 61-53032
(patent reference 3) Japanese Unexamined Patent Publication (Kokai) No. 11-318433

### DISCLOSURE OF THE INVENTION

An object of the present invention is to provide a process for producing mycelia of a Matsutake fungus (Matsutake mycelia) useful as a seed culture for a large-scale production of Matsutake mycelia, and a process for producing a large number of Matsutake mycelia, using the seed culture, without a loss of physiological activities.

The present inventors have conducted intensive studies and, as a result, found that a seed culture for a large-scale production of Tricholoma matsutake mycelia can be obtained by cultivating Tricholoma matsutake mycelia on a small scale under agitation without aeration or with aeration at a low rate in a liquid medium. Preferably, the Tricholoma matsutake mycelia used in the agitation cultivation is produced by cultivating initial mycelia (previously cultivated or maintained in a solid or liquid medium) in a liquid medium under stationary conditions for an appropriate period, and further cultivating the obtained mycelia under shaking. Further, the present inventors found that a large number of Tricholoma matsutake mycelia can be efficiently produced by cultivating the seed culture under submerged conditions (for example, submerged cultivation under agitation).

The present invention relates to:
[1] a process for producing a mycelium of a Matsutake fungus, comprising the step of cultivating a mycelium on a small scale under agitation without aeration or with aeration at a low rate of less than 0.05 vvm in a liquid medium (hereinafter referred to as agitating cultivation without aeration);
[2] the process of [1], further comprising, as a precultivation step before the agitating cultivation step,
   (1) cultivating a mycelium in a liquid medium under stationary conditions,
   (2) cultivating a mycelium under shaking, or
   (3) cultivating a mycelium in a liquid medium under stationary conditions, and further cultivating the obtained mycelium under shaking;
[3] the process of [1] or [2], wherein an agitation power per unit volume of a culture medium in the agitating cultivation step is 0.01 to 2 kW/m³;
[4] a process for producing a mycelium of a Matsutake fungus (particularly, a seed culture for producing a mycelium of a Matsutake fungus), comprising the steps of:
   cultivating a mycelium in a liquid medium under stationary conditions, and
   cultivating the obtained mycelium under shaking;
[5] a process for producing a mycelium of a Matsutake fungus, comprising the step of cultivating the mycelium obtained by the process of any one of [1] to [4] as a seed culture under submerged conditions;
[6] the process of any one of [2] to [5], wherein a period of the stationary cultivation step is 30 to 400 days;
[7] the process of any one of [2] to [6], wherein a period of the shaking cultivation step is 5 to 50 days;
[8] the process of any one of [1] to [7], wherein a propagation rate in inoculation is 2 to 50 times;
[9] the process of any one of [1] to [8], wherein an osmotic pressure of a culture medium is 0.01 to 0.8 MPa; and
[10] the process of any one of claim [1] to [9], wherein a concentration of a fibrous mycelium contained in an initial mycelium is 0.05 g/L or more.

According to a preferred embodiment of the submerged cultivation step in the process of [5], the mycelium of a Matsutake fungus produced by the process of any one of [1] to [3] is used as the seed culture to carry out submerged cultivation on a large scale.

Further, the present invention relates to a process for producing a mycelium of a Matsutake fungus, comprising the steps of:
cultivating a mycelium cultivated or maintained in a solid or liquid medium, in a liquid medium under stationary conditions, and
cultivating the obtained mycelium under shaking.

Further, the present invention relates to a process for producing a mycelium of a Matsutake fungus, comprising the steps of:
cultivating a mycelium cultivated or maintained in a solid or liquid medium, in a liquid medium under stationary conditions,
cultivating the obtained mycelium under shaking, and
carrying out an agitating cultivation without aeration in a liquid medium using a small-sized fermentor of less than 100 L.

Further, the present invention relates to a process for producing a mycelium of a Matsutake fungus, comprising the steps of:
cultivating a mycelium cultivated or maintained in a solid or liquid medium, in a liquid medium under stationary conditions,
cultivating the obtained mycelium under shaking,
carrying out agitating cultivation without aeration in a liquid medium using a small-sized fermentor of less than 100 L to produce a seed culture, and
cultivating the obtained seed culture under submerged conditions using a middle-sized or large-sized fermentor of 100 L or more.

The terms "small-scale cultivation or production" and "large-scale cultivation or production" as used herein are well-known meanings used in well-known stepwise cultivation methods for producing a large number of microorganisms, in which the initial cultivation is started with a small-scale culture medium, and stepwisely transferred to cultivations with a large-scale culture medium. Therefore, each range of "small-scale" or "large-scale" is not absolutely defined by a specific volume of culture medium, but is a relative concept which can be appropriately determined in accordance with, for example, scale-up procedures (particularly a volume of a fermentor).

Similarly, the terms "small-sized fermentor" and "large-sized fermentor" as used herein are not absolutely defined by specific volumes thereof, but are relative concepts which can be appropriately determined in accordance with, for example, scale-up procedures (particularly a scale of cultivation).

In this connection, preferable ranges of the terms will be illustrated in detail hereinafter.

The following expressions will be used hereinafter to facilitate an understanding of the present invention:

The initial strain of a Matsutake fungus is referred to as Matsutake I.

Mycelia of a Matsutake fungus obtained by cultivating or maintaining the Matsutake I in a solid or liquid medium are referred to as Matsutake II.

Mycelia of a Matsutake fungus obtained by cultivating the Matsutake II in a liquid medium under stationary conditions are referred to as Matsutake III.

Mycelia of a Matsutake fungus obtained by cultivating the Matsutake III under shaking are referred to as Matsutake IV.

Mycelia of a Matsutake fungus obtained by cultivating the Matsutake IV on a small scale under agitation without aeration or with aeration at a low rate in a liquid medium using a small-sized fermentor (for example, a small-sized fermentor having a volume of less than 100 L) are referred to as Matsutake V.

Mycelia of a Matsutake fungus obtained by cultivating the Matsutake V under submerged conditions on a large scale or using a large-sized fermentor (for example, a large-sized fermentor having a volume of 100 L or more) are referred to as Matsutake VI.

Mycelia of a Matsutake fungus obtained by cultivating the Matsutake VI under submerged conditions on a large scale or using a large-sized fermentor (for example, a large-sized fermentor having a volume of 100 L or more) are referred to as Matsutake VII.

Mycelia of a Matsutake fungus obtained by cultivating the Matsutake VII under submerged conditions on a large scale or using a large-sized fermentor (for example, a large-sized fermentor having a volume of 100 L or more) are referred to as Matsutake VIII.

### BRIEF DESCRIPTION OF THE DRAWINGS

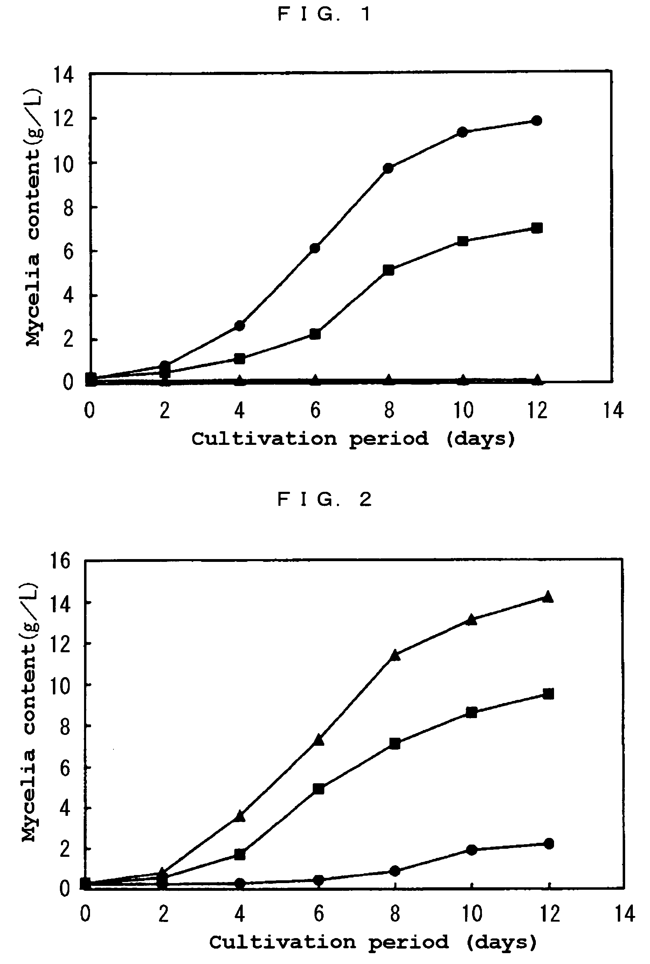

Figure 1 is a graph showing the relationship between an agitation power and growth when the cultivation is carried out at 23±1°C using a 200-L fermentor and a medium culture containing starch, glucose, potassium dihydrogen phosphate, and the like. The symbols "solid circle", "solid square", and "solid triangle" indicate the results at the agitation powers of 0.12 kw/m³, 1.1 kw/m³, and 2.6 kw/m³, respectively.
Figure 2 is a graph showing the relationship between an osmotic pressure and growth when the cultivation is carried out at 23±1°C using a 200-L fermentor (agitation power = 0.12 kw/m³) and a medium culture containing starch, glucose, potassium dihydrogen phosphate, and the like. The symbols "solid circle", "solid square", and "solid triangle" indicate the results at the osmotic pressures of 0.98 MPa, 0.5 MPa, and 0.05 MPa, respectively.

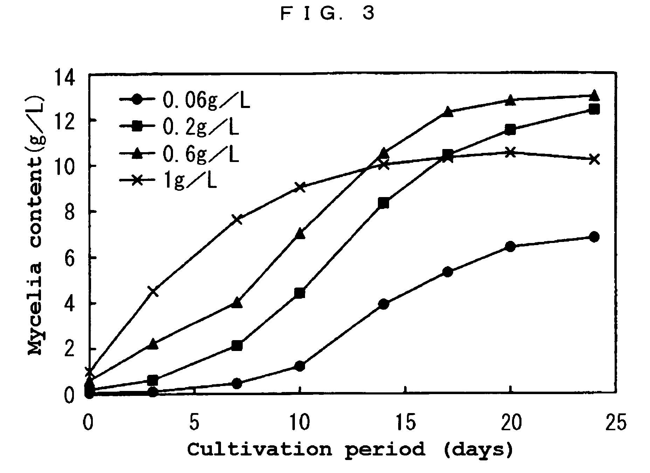

Figure 3 is a graph showing the relationship between a concentration of initial mycelia and growth when the cultivation is carried out at 23±1°C using a 500-mL flask with agitation (agitation power = 0.14 kw/m³) and a medium culture containing starch, glucose, potassium dihydrogen phosphate, and the like. The symbols "solid circle", "solid square", "solid triangle", and "X" indicate the results at the concentrations of initial mycelia of 0.06 g/L, 0.2 g/L, 0.6 g/L, and 1 g/L, respectively.

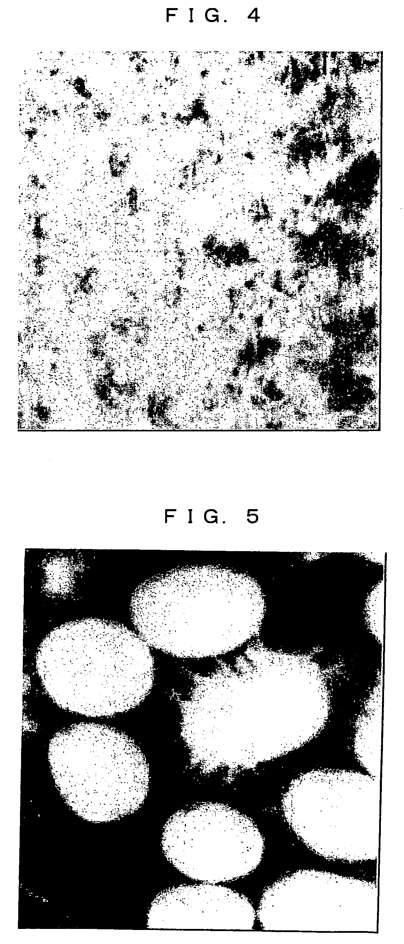

Figure 4 shows a form of fibrous mycelia.
Figure 5 shows a form of pelleted mycelia.

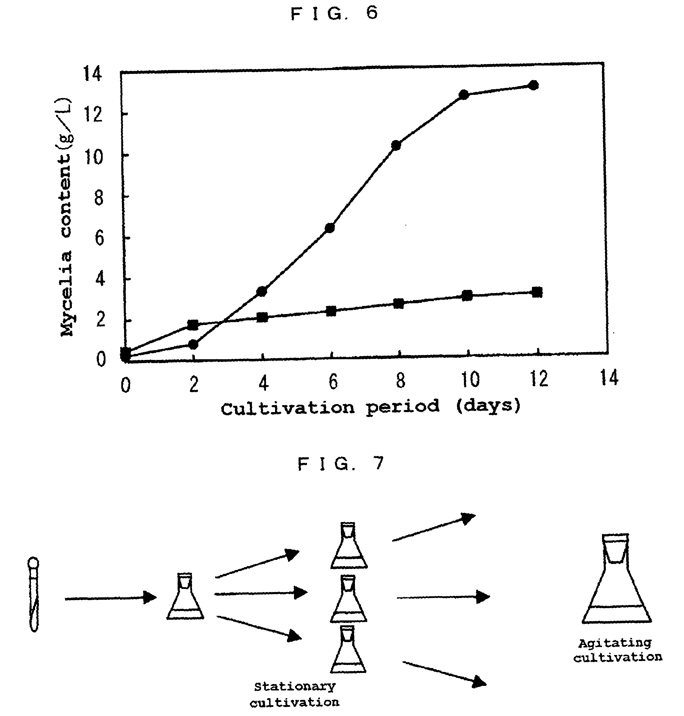

Figure 6 is a graph showing the relationship between a form of a culture seed and growth when the cultivation is carried out at 23±1°C using a 200-L fermentor (agitation power = 0.12 kw/m³; volume of culture medium = 140 L) and a medium culture containing starch, glucose, potassium dihydrogen phosphate, and the like. The symbols "solid circle" and "solid square" indicate the results when the seed cultures were fibrous mycelia and pelleted mycelia, respectively.
Figure 7 shows the outline of a cultivation system for a seed culture.

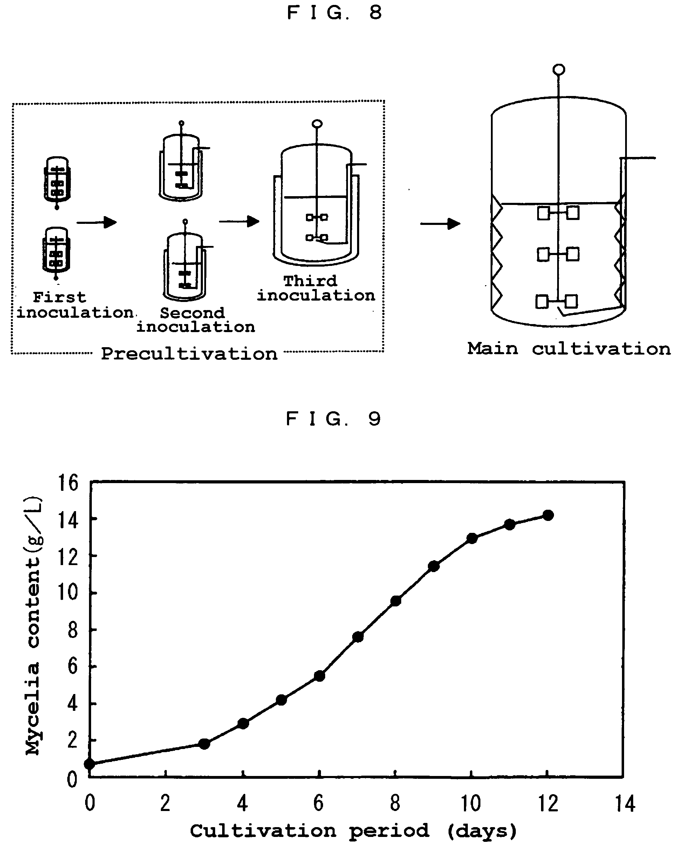

Figure 8 shows a cultivation system capable of carrying out the process of the present invention.
Figure 9 is a graph showing the relationship between a cultivation period and a mycelia content when the cultivation is carried out at 23±1°C using a 65-m³ fermentor (agitation power = 0.01 to 0.12 kw/m³; volume of culture medium = 40 m³) and a medium culture containing starch, glucose, potassium dihydrogen phosphate, and the like.

### BEST MODE FOR CARRYING OUT THE INVENTION

In the process of the present invention, mycelia of a Matsutake fungus can be produced by carrying out at least the agitating cultivation step without aeration. The term "agitating cultivation without aeration" as used herein means that a cultivation without aeration or with aeration thereto at a rate of 0.05 vvm or less (i.e., without substantial aeration) in a liquid phase (i.e., a liquid medium) under agitating. In this connection, the term "agitating cultivation without aeration" includes a case in which aeration in a gas phase is carried out to maintain an internal pressure of a fermentor. Mycelia of a Matsutake fungus produced by the agitating cultivation step without aeration may be a final product, or may be used as a seed culture for a large-scale production. It is preferable to use the mycelia as the seed culture for a large-scale production.

As mycelia of a Matsutake fungus (Matsutake mycelia) which may be used in the agitating cultivation step without aeration in the process of the present invention, there may be mentioned, for example, Matsutake mycelia obtained by carrying out an appropriate precultivation for the purpose of, for example, growth or acclimation. As the precultivation, there may be mentioned, for example, stationary liquid cultivation or shaking cultivation, or a combination thereof (particularly, successive cultivation of stationary liquid cultivation and shaking cultivation).

Hereinafter, the process of the present invention will be explained in the sequential order of stationary liquid cultivation, shaking cultivation, agitating cultivation without aeration, and submerged cultivation.

The term "Matsutake fungus" as used herein means a fungus belonging to genus Tricholoma, including Tricholoma matsutake and relatives thereof, such as Tricholoma fulvocastaneum Hongo sp.nov., Tricholoma bakamatsutake Hongo sp.nov., or Tricholoma robustum Ricken.

As a Matsutake fungus (for example, Matsutake I as the initial strain of a Matsutake fungus) used in the process of the present invention, mycelia isolated from a naturally-occurring or commercially available fruit body may be used.

Further, a strain commercially available or deposited in, for example, the International Patent Organism Depositary National Institute of Advanced Industrial Science and Technology may be used in the process of the present invention. As the strain, there may be mentioned, for example, strains ATCC34979, ATCC34981, and ATCC34988 deposited in American Type Culture Collection (ATCC), strains IFO6915, IFO6925, IFO6930, IFO6935, IFO30604, IFO30605, and IFO30606 deposited in Institute for Fermentation, Osaka (IFO), strain MAFF460038 deposited in National Institute of Agrobiological Sciences, and Tricholoma matsutake strain FERM BP-7304 deposited in the International Patent Organism Depositary National Institute of Advanced Industrial Science and Technology.

A medium used in cultivating or maintaining Matsutake II is not particularly limited, so long as it contains the substrates for nutrient source commonly used in cultivating a Matsutake fungus. As the medium, there may be mentioned, for example, Ohta medium [Ohta, A., (1990) Trans. Mycol. Soc. Japan 31: 323-334], MMN medium [Marx, D.H. (1969) Phytopathology 59: 153-163], or Hamada medium [Hamada, (1964) Matsutake, 97-100].

As an agent for solidifying a solid medium, there may be mentioned, for example, carageenan, mannan, pectin, agar, curdlan, starch, or alginate. Agar is preferable.

As the substrates for nutrient source, for example, carbon sources, nitrogen sources, or inorganic element sources may be used.

As the carbon sources, there may be mentioned, for example, starch (such as rice starch, wheat starch, potato starch, or sweet potato starch), polysaccharides (such as dextran or amylopectin), oligosaccharides (such as maltose or sucrose), monosaccharides (such as fructose or glucose), or malt extract. There is a period in which monosaccharides such as glucose is preferable and a period in which starch is preferable, in accordance with a growth rate of a Matsutake fungus. Therefore, one or more appropriate carbon sources may be selected in accordance with such periods, and, if necessary, a combination thereof can be used.

As the nitrogen sources, there may be mentioned, for example, yeast extract, dried yeast, corn steep liquor, soybean powder, or soybean peptone, which are derived from natural substances. Further, ammonium nitrate, ammonium sulfate, or urea may be used. The above nitrogen sources may be used alone or in a combination thereof. Substances derived from natural substances (particularly yeast extract) are generally preferable in the light of a growth rate.

The inorganic element sources are used to supply phosphates and trace elements. There may be mentioned, for example, phosphates or inorganic salts such as sulfates, hydrochlorides, nitrates, or phosphates of metal ions (for example, sodium, potassium, magnesium, calcium, zinc, manganese, copper, or iron). The required amount is dissolved in a culture medium.

Further, vitamins such as vitamin B1 or amino acids may be added to a culture medium.

Furthermore, for example, plant extracts, organic acids, or nucleic acid related substances may be added in accordance with the properties of a Matsutake fungus used. As the plant extracts, there may be mentioned, for example, extracts of fruit vegetables, root vegetables, or leaf vegetables. As the organic acids, there may be mentioned, for example, citric acid, tartaric acid, malic acid, fumaric acid, or lactic acid. As the nucleic acid related substances, there may be mentioned, for example, commercially available nucleic acid, nucleic acid extract, yeast, or yeast extract.

When a solid medium is prepared, the content of the carbon sources is preferably 10 to 100 g/L, more preferably 10 to 50 g/L, most preferably 20 to 30 g/L.

The content of the nitrogen sources (as the amount of the nitrogen element converted) is preferably 0.005 to 0.1 mol/L, more preferably 0.007 to 0.07 mol/L, most preferably 0.01 to 0.05 mol/L.

The content of phosphates (as the amount of the phosphorus element converted) is preferably 0.001 to 0.05 mol/L, more preferably 0.005 to 0.03 mol/L, most preferably 0.01 to 0.02 mol/L. The other inorganic salts, vitamins, plant extracts, organic acids, and/or nucleic acid related substances may be appropriately added in accordance with the properties of a Matsutake fungus. Further, the pH of a prepared solution containing substances for nutrient source is preferably pH 4 to 7, more preferably pH 4.5 to 6.0, most preferably pH 5.0 to 5.5.

### (Stationary liquid cultivation)

The process for producing Matsutake III by cultivating Matsutake II (Matsutake fungus cultivated or maintained in a solid or liquid medium) in a liquid medium under stationary conditions will be explained.

In the process, a culture vessel (for example, a 30-mL to 10-L conical flask, preferably a 100-mL to 2-L conical flask) capable of carrying out a stationary liquid cultivation is generally used.

The stationary liquid cultivation is started by inoculating a liquid medium with Matsutake II.

As to a volume of the liquid medium, a ratio (Vt/Vo; hereinafter referred to as "propagation rate in inoculation") of a total volume (Vt) of a broth containing Matsutake II for inoculation and the liquid medium with respect to a volume of the broth (Vo) is preferably 2 to 50 times, more preferably 3 to 30 times.

In the inoculation of the liquid medium with the broth containing Matsutake II, a ratio (Wo/Vt; hereinafter referred to as "concentration of initial mycelia") of a dried weight (Wo) of mycelia of Matsutake II contained in the broth containing Matsutake II for inoculation with respect to a volume (Vt) of the mixture of the broth and the liquid medium is preferably 0.05 to 3 g/L, more preferably 0.1 to 2 g/L.

In the stationary liquid cultivation, the cultivation is carried out at preferably 15 to 30°C, more preferably 20 to 25°C for preferably 30 to 400 days, more preferably 120 to 240 days. When the period for the stationary liquid cultivation is less than 30 days or more than 400 days, it becomes difficult to obtain Matsutake III having a viability suitable for a large-scale cultivation.

The liquid medium used in the stationary liquid cultivation contains substrates for nutrient source, and thus an osmotic pressure of the liquid medium is preferably 0.01 to 0.8 MPa, more preferably 0.02 to 0.7 MPa, most preferably 0.03 to 0.5 MPa.

As the substrates for nutrient source used in the stationary liquid cultivation, the same carbon sources, nitrogen sources, inorganic element sources, vitamins (such as vitamin B1), or amino acids as the above-mentioned solid medium for cultivating Matsutake I can be used.

The content of the carbon sources is preferably 10 to 100 g/L, more preferably 20 to 60 g/L, most preferably 25 to 45 g/L. Monosaccharides such as glucose are generally used.

The content of the nitrogen sources (as the amount of the nitrogen element converted) is preferably 0.005 to 0.1 mol/L, more preferably 0.007 to 0.07 mol/L, most preferably 0.01 to 0.05 mol/L.

When phosphates are used, the content thereof (as the amount of the phosphorus element converted) is preferably 0.001 to 0.05 mol/L, more preferably 0.005 to 0.03 mol/L, most preferably 0.01 to 0.02 mol/L.

The other inorganic salts, vitamins, plant extracts, organic acids, and nucleic acid related substances may be appropriately added in accordance with the properties of a Matsutake fungus.

Further, the pH of a prepared solution containing substances for nutrient source is preferably pH 4 to 7, more preferably pH 4.5 to 6.5, most preferably pH 5.0 to 6.0.

In the stationary liquid cultivation, the starting culture medium can be inoculated with a portion or the whole of the Matsutake III-containing broth previously obtained by another stationary liquid cultivation, as well as with the Matsutake II-containing broth as described above.

### (Shaking cultivation)

The process for producing Matsutake IV by cultivating Matsutake III under shaking will be explained.

In the process, a culture vessel (for example, a 30-mL to 10-L, preferably a 300-mL to 5-L conical flask or shaking flask capable of carrying out shaking cultivation is generally used.

The shaking cultivation can be started by inoculating a liquid medium with Matsutake III (i.e., Matsutake fungus obtained by the stationary liquid cultivation), or with Matsutake II (i.e., Matsutake fungus cultivated or maintained in a solid or liquid medium). Before the inoculation, it is preferable to homogenize the Matsutake mycelia (i.e., Matsutake III or Matsutake II) used for inoculation.

As to a volume of the liquid medium, a ratio (hereinafter referred to as "propagation rate in inoculation") of a total volume of a broth containing Matsutake III for inoculation and the liquid medium with respect to a volume of the broth is preferably 2 to 50 times, more preferably 3 to 30 times, most preferably 5 to 10 times.

In this connection, the stationary liquid cultivation may be carried out using plural culture vessels to prepare a sufficient amount of broth required for an appropriate propagation rate in inoculation.

In the inoculation of the liquid medium with the broth containing Matsutake III, a ratio (hereinafter referred to as "concentration of initial mycelia") of a dried weight of mycelia of Matsutake III contained in the broth containing Matsutake III for inoculation with respect to a volume of the mixture of the broth and the liquid medium is preferably 0.05 to 3 g/L, more preferably 0.1 to 2 g/L.

The shaking cultivation is carried out at preferably 15 to 30°C, more preferably 20 to 25°C for preferably 5 to 50 days, more preferably 7 to 50 days, most preferably 14 to 28 days to produce Matsutake IV used for the agitating cultivation.

In the shaking cultivation, an agitation power per unit volume of a culture medium contained in a conical flask is generally 0.01 to 2 kW/m³, preferably 0.05 to 0.4 kW/m³.

The liquid medium used in the shaking cultivation contains substrates for nutrient source, and thus an osmotic pressure of the liquid medium is preferably 0.01 to 0.8 MPa, more preferably 0.02 to 0.7 MPa, most preferably 0.03 to 0.5 MPa.

As the substrates for nutrient source used in the shaking cultivation, the same carbon sources, nitrogen sources, inorganic element sources, vitamins (such as vitamin B1), or amino acids as the above-mentioned liquid medium for cultivating Matsutake II can be used.

The content of the carbon sources is preferably 10 to 100 g/L, more preferably 20 to 60 g/L, most preferably 25 to 45 g/L. Monosaccharides such as glucose are generally used.

The content of the nitrogen sources (as the amount of the nitrogen element converted) is preferably 0.005 to 0.1 mol/L, more preferably 0.007 to 0.07 mol/L, most preferably 0.01 to 0.05 mol/L.

The content of phosphates (as the amount of the phosphorus element converted) is preferably 0.001 to 0.05 mol/L, more preferably 0.005 to 0.03 mol/L, most preferably 0.01 to 0.02 mol/L.

The other inorganic salts, vitamins, amino acids, plant extracts, organic acids, and/or nucleic acid related substances may be appropriately added in accordance with the properties of a Matsutake fungus.

Further, the pH of a prepared solution containing substances for nutrient source is preferably pH 4 to 7, more preferably pH 4.5 to 6.5, most preferably pH 5.0 to 6.0.

### (Agitating cultivation without aeration)

The process for producing Matsutake V by agitating cultivation without aeration will be explained.

The agitating cultivation is started by inoculating a liquid medium with Matsutake IV (i.e., Matsutake fungus obtained by the shaking cultivation) or with Matsutake III (i.e., Matsutake fungus obtained by the stationary liquid cultivation). Before the inoculation, the Matsutake mycelia (i.e., Matsutake III or Matsutake II) used for inoculation can be homogenized.

Hereinafter, the agitating cultivation step without aeration will be explained by an embodiment in which the liquid medium is inoculated with Matsutake IV, but an embodiment in which the liquid medium is inoculated with Matsutake III can be similarly carried out. Further, mycelia of a Matsutake fungus obtained by the agitating cultivation can be used to repeat the agitating cultivation.

The liquid medium used in the agitating cultivation without aeration may be prepared as follows:

As the substrates for nutrient source, the same carbon sources, nitrogen sources, inorganic element sources, vitamins (such as vitamin B1), or amino acids as the above-mentioned liquid medium for shaking cultivation can be used.

The content of the carbon sources is preferably 10 to 100 g/L, more preferably 20 to 60 g/L, most preferably 25 to 45 g/L. Starch may be preferably used.

When monosaccharides (such as glucose), which affect an osmotic pressure of the liquid medium for cultivating under agitation without aeration, are used together with starch, the content of the monosaccharides is preferably 0.1 to 60 g/L, more preferably 0.5 to 40 g/L, most preferably 0.7 to 20 g/L.

The content of the nitrogen sources (as the amount of the nitrogen element converted) is preferably 0.005 to 0.1 mol/L, more preferably 0.007 to 0.07 mol/L, most preferably 0.01 to 0.05 mol/L.

The content of phosphates (as the amount of the phosphorus element converted) is preferably 0.001 to 0.05 mol/L, more preferably 0.005 to 0.03 mol/L, most preferably 0.01 to 0.02 mol/L.

The other inorganic salts, vitamins, amino acids, plant extracts, organic acids, and/or nucleic acid related substances may be appropriately added in accordance with the properties of a Matsutake fungus.

Further, the pH of a prepared solution containing substances for nutrient source is preferably pH 4 to 7, more preferably pH 4.5 to 6.5, most preferably pH 5.0 to 6.0.

The liquid medium used in the agitating cultivation without aeration contains substrates for nutrient source, and thus an osmotic pressure of the liquid medium is preferably 0.01 to 0.8 MPa, more preferably 0.02 to 0.7 MPa, most preferably 0.03 to 0.5 MPa.

The temperature in the agitating cultivation without aeration is preferably 15 to 30°C, more preferably 20 to 25°C.

As to a volume of the liquid medium, a ratio (hereinafter referred to as "propagation rate in inoculation") of a total volume of a broth containing Matsutake IV for inoculation and the liquid medium with respect to a volume of the broth is preferably 2 to 50 times, more preferably 3 to 30 times, most preferably 5 to 10 times.

In the inoculation of the liquid medium with the broth containing Matsutake IV, a ratio (hereinafter referred to as "concentration of initial mycelia") of a dried weight of mycelia of Matsutake IV contained in the broth containing Matsutake IV for inoculation with respect to a volume of the mixture of the broth and the liquid medium is preferably 0.01 to 5 g/L, more preferably 0.05 to 3 g/L, most preferably 0.1 to 2 g/L. In this connection, it is preferable that a concentration of fibrous mycelia contained in the initial mycelium is 0.005 to 5 g/L, more preferably 0.025 to 3 g/L, most preferably 0.05 to 2 g/L.

When Matsutake V obtained in the agitating cultivation without aeration are used as a seed culture for the following submerged cultivation, a period of the agitating cultivation without aeration is preferably 3 to 20 days, more preferably 5 to 14 days.

The broth in which a dried weight of mycelia of Matsutake V is preferably 0.5 to 10 g/L, more preferably 1 to 8 g/L, most preferably 1 to 6 g/L after the above cultivation period contains Matsutake V having a viability suitable for submerged cultivation.

When Matsutake V obtained in the agitating cultivation without aeration are separated as a final product, a period of the agitating cultivation without aeration is preferably 5 to 30 days, more preferably 7 to 20 days, most preferably 10 to 15 days.

After the above cultivation period, the cultivation can be finished, preferably when a rate of utilizing carbon sources is remarkably decreased. The cultivation period can be appropriately selected in accordance with a production process including a production cycle or cost.

In the shaking cultivation and the subsequent cultivations, the obtained mycelia are broadly classified into fibrous mycelia and pelleted mycelia.

In the process of the present invention, when mycelia produced in each step is used as a seed culture for the subsequent step, mycelia containing 50% or more (more preferably 80% or more) of fibrous mycelia is preferable. When a liquid medium is inoculated with fibrous mycelia, a rapid growth is observed, in comparison with pelleted mycelia. In this connection, when a sufficient amount of the whole mycelia can be prepared, even if the content of fibrous mycelia contained in the whole mycelia is less than the above percentage, a sufficient amount of fibrous mycelia is contained therein, and thus, the percentage of fibrous mycelia is not particularly limited. The ratio of fibrous mycelia to pelleted mycelia may be determined, for example, by passing the mycelia mixture through a mesh filter having a mesh opening of approximately 1 mm.

In contrast, when mycelia obtained in a certain step is separated as a final product, the ratio of fibrous mycelia to pelleted mycelia after the final step is not important and is not particularly limited, because it is important to collect a sufficient amount of mycelia as the whole.

The agitating cultivation without aeration is carried out on a small scale, i.e., using a small-scale culture medium. A volume of the medium used in the agitating cultivation without aeration is not particularly limited, so long as it does not exceed a range of a small-scale cultivation in an ordinary stepwise cultivation for producing a large number of microorganisms. A volume of the medium used in the agitating cultivation without aeration is generally less than 1000 L, preferably less than 500 L, more preferably less than 100 L. The lower limit of the medium is generally 0.8 L or more, preferably 4 L or more.

In the agitating cultivation without aeration, for example, a fermentor capable of accomplishing sterility and capable of carrying out a small-scale cultivation may be used. As such a fermentor, a small-sized fermentor (such as a jar fermentor or a small-sized culture tank) having a capacity of, for example, 1000 L or less, preferably 500 L or less, more preferably 100 L or less, most preferably less than 100 L, may be used. The lower limit of the capacity is generally 1 L or more, preferably 5 L or more.

When Matsutake V is produced by cultivating Matsutake IV, an agitating cultivation is carried out without aeration in the liquid medium. When cultivation on a small scale (for example, using a jar fermentor or small-sized culture tank having a capacity of less than 100 L) is carried out with aeration, mycelia sometimes flocculate and lose growth points, and thus, sometimes lose viability as a seed culture.

In the initial stage of the agitating cultivation without aeration, the agitation is controlled by a agitation power per unit volume of a culture medium. Mycelia exhibit a rapid growth under agitation at generally 0.01 to 2 kW/m³, preferably 0.05 to 1 kW/m³. During the period subsequent to the initial stage, an oxygen supply is decreased by the growth of mycelia, and it becomes hard to disperse growing mycelia, and thus, it is necessary to appropriately increase a strength of the agitation.

Mycelia produced by the agitating cultivation without aeration may be separated and collected in accordance with a conventional method, such as filtration with a filter press, or centrifugation. The obtained mycelia can be dried (or not dried), crushed, extracted, or formed in accordance with the intended purpose as a final product.

### (Submerged cultivation)

The submerged cultivation may be started by inoculating a liquid medium with Matsutake V (i.e., Matsutake fungus obtained by the agitating cultivation without aeration). Further, mycelia of a Matsutake fungus (for example, Matsutake V to Matsutake VII) obtained by the submerged cultivation can be used to repeat the submerged cultivation. Furthermore, Matsutake IV [i.e., Matsutake fungus obtained by the shaking cultivation (particularly, Matsutake fungus obtained by the stationary liquid cultivation, followed by the shaking cultivation)] can be used in the submerged cultivation. Before the inoculation, the Matsutake mycelia used for inoculation can be homogenized.

In the submerged cultivation, air is forcefully supplied from the outside of a fermentor to the culture medium. In addition, the air bubbles supplied to the medium are changed to microbubbles, by mechanical agitation with an agitator, or by a draft tube or a plate for dispersion, to enlarge a gas/liquid interface and prolong a residence time of the bubbles in the culture medium. As a result, an efficient oxygen supply to microorganisms is accomplished in the submerged cultivation. The submerged cultivation may be carried out using, for example, an aeration stirred fermentor, a bubble tower fermentor (an airlift fermentor), or a fluidized bed fermentor.

As the liquid medium used in the submerged cultivation, the same liquid medium as that used in the agitating cultivation without aeration may be used. Further, the same substrates as used in the agitating cultivation without aeration may be used, and the concentrations of the substrates may be adjusted in accordance with a desired yield. It is preferable to adjust an osmotic pressure derived from the substrates to 0.01 to 0.8 MPa.

With respect to the temperature in cultivation, the propagation rate in inoculation, the concentration of initial mycelia, the period for cultivation, and the agitation power, the above explanations of the agitating cultivation without aeration are applicable to the submerged cultivation.

It is preferable that the submerged cultivation is carried out on a large scale, i.e., by using a large-scale culture medium. A volume of the medium used in the submerged cultivation is not particularly limited, so long as it does not exceed a range of a large-scale cultivation in an ordinary stepwise cultivation for producing a large number of microorganisms. A volume of the medium used in the submerged cultivation is generally 100 L or more, preferably 1000 L or more, more preferably 3000 L or more.

In the submerged cultivation, for example, a fermentor capable of accomplishing sterility and capable of carrying out aeration (if necessary), submerged cultivation, and a large-scale cultivation may be used. As such a fermentor, a large-sized fermentor having a capacity of, for example, 100 L or more, preferably 1000 L or more, more preferably 3000 L or more, may be used. In this connection, the term "large-sized fermentor" as used herein includes a fermentor sometimes referred to as a "middle-sized fermentor".

When the submerged cultivation on a large scale is carried out on an industrial scale, for example, by using a large-sized fermentor having an capacity of 100 L or more, aeration is carried out, if necessary, at a rate of preferably 0.05 to 1.0 vvm, more preferably 0.2 to 0.5 vvm.

In the initial stage of the submerged cultivation, the agitation is controlled by a agitation power per unit volume of a culture medium. Mycelia exhibit a rapid growth under agitation at generally 0.01 to 2 kW/m³, preferably 0.05 to 1 kW/m³. During the period subsequent to the initial stage, an oxygen supply is decreased by the growth of mycelia, and it becomes hard to disperse growing mycelia, and thus, it is necessary to appropriately increase a strength of the agitation. In the submerged cultivation, preferably the cultivation at the initial stage is carried out with aeration at a low rate and under agitation at a low rate, and the cultivation at the late stage is carried out with aeration at a high rate and under agitation at a high rate.

Mycelia produced by the submerged cultivation may be separated and collected in accordance with a conventional method, such as filtration with a filter press, or centrifugation. The obtained mycelia can be dried (or not dried), crushed, extracted, or formed in accordance with the intended purpose as a final product.

Hereinafter the present invention will be further explained by an embodiment using Tricholoma matsutake FERM BP-7304. The following explanations are generally applicable to Tricholoma matsutake strains other than the FERM BP-7304 strain.

### 1. Properties in growth

The FERM BP-7304 strain is sensitive to environmental factors. Physical environmental factors include, for example, an osmotic pressure, or a mechanical shock due to agitation. Because the growth requires a long time, a large number of mycelia as a seed culture are needed in each inoculation to finish the cultivation for short periods. Further, it is necessary to establish a cultivation system capable of mass inoculation.

### 2. Basic cultivation conditions

### (1) Resistance to mechanical shock

The main purpose of agitation in agitating cultivation with aeration is a dispersion of oxygen and nutrients. A growth rate of the FERM BP-7304 strain is slow, and a demand for oxygen is small, and thus, an oversupply of oxygen is unnecessary. Figure 1 is a graph showing the relationship between an agitation power and growth. When the agitation power is strong, the growth of mycelia is inhibited.
Because a decrease in dissolved oxygen is not observed, the main effect due to the agitation is considered a mechanical shock.

In addition, when the agitation power is strong, mycelia tend to become pelleted, and thus, the growth is retarded. It is considered that the results are caused by a flocculation effect by agitation. The mechanical damage to mycelia and the transformation into a pelleted form are important factors in the growth of the FERM BP-7304 strain.

### (2) Growth and osmotic pressure

In the passway for utilizing carbon sources in basidiomycetes, it is generally considered that saccharides is decomposed into glucose, and further, glucose is converted into glucose-6-phosphate, and then glucose-6-phosphate is incorporated into cells. That is, monosaccharides such as glucose are generally superior in efficiency to polysaccharides. In the FERM BP-7304 strain, it is considered that polysaccharides are utilized via glucose, but growth tends to be inhibited in a culture medium containing a high concentration of glucose and exhibiting a high osmotic pressure. Figure 2 shows the results of cultivation using culture media containing starch and glucose and exhibiting various osmotic pressures. When the concentration of glucose is high, the growth rate is slow, and the result suggests that the resistance to an osmotic pressure is low. When preparing a culture medium, a small amount of glucose is added to a medium containing starch as a main carbon source, to fully exploit the advantageous properties of two carbon sources.

### (3) Required amount of seed culture

It is known that Matsutake fungi grow slowly.
Similarly, the FERM BP-7304 strain tends to grow slowly, but in an industrial production the growth should be finished by a predetermined period. Figure 3 shows the relationship between a growth rate of the FERM BP-7304 strain and the amount of seed culture for inoculation. When the concentration of initial mycelia is approximately 0.1 to 2.0 g/L (including 0.05 to 2.0 g/L of fibrous mycelia), the growth is rapid, and thus it is suggested that mass inoculation is necessary in an industrial cultivation. Particularly, when the amount of inoculation is small, the industrial cultivation tends to be difficult because a remarkably long induction period is required.

### (4) Control of mycelia form

In the cultivation of filamentous fungi it is known that the form of mycelia significantly influences the growth. The FERM BP-7304 strain is broadly classified into fibrous mycelia (Figure 4) and pelleted mycelia (Figure 5), and the fibrous mycelia are advantageous to the growth. Figure 6 shows growth curves in which pelleted and fibrous seed cultures are used for inoculation. When the pelleted seed culture was used for inoculation, the growth rate tends to be remarkably slow. Therefore, it is important in the industrial production to control the form of the mycelia.
As factors contributing to the form of the FERM BP-7304 strain, there may be mentioned, for example, the flow of a culture medium, the amount of seed culture for inoculation, or the composition of a culture medium. Particularly, in a small-sized jar fermentor, mycelia tend to be entangled and fused with each other to become pelleted, due to the flow of a culture medium by aeration. Therefore, an optimization of aeration is preferable to obtain an excellent seed culture. Because pelleted mycelia do not grow efficiently as a seed culture, the content of fibrous mycelia contained in a seed culture for industrial cultivation is preferably 50% ore more, most preferably 80%. In this connection, if the initial mycelia in the next stage contains approximately 0.05 to 2 g/L (as dried weight) of fibrous mycelia, the content of fibrous mycelia contained in a seed culture is not limited to the above range.

### 3. Cultivation system

### (1) Cultivation system for seed culture

As described above, the FERM BP-7304 strain tends to need a large amount of seed culture, and thus, it is preferable to use a system capable of cultivating a large amount of seed culture. Therefore, the present inventors established a method for cultivating a seed culture comprising the steps of cultivating a seed culture maintained on a solid medium under stationary conditions stepwisely, and carrying out shaking cultivation, as shown in Figure 7. The purpose of the cultivation is acclimation to a liquid medium.

### (2) Tank cultivation system

As described above, the FERM BP-7304 strain tends to need a large amount of seed culture for a rapid growth of the FERM BP-7304 strain. Therefore, the concentration of initial mycelia in a tank cultivation system is preferably approximately 0.1 to 2.0 g/L. Further, mycelia in the initial culture medium contain preferably approximately 0.05 to 2.0 g/L (as the concentration of initial mycelia) of fibrous mycelia. Figure 8 shows a cultivation system capable of carrying out the process of the present invention. In the tank cultivation system, four stages of cultivation including the main cultivation stage may be carrying out. In each stage, the propagation rate is set at approximately 5 to 15 times to obtain approximately 0.1 to 2.0 g/L of the concentration of initial mycelia. According to the cultivation system and method shown in Figure 8, approximately 12 to 14 g/L of mycelia can be produced by carrying out the main cultivation for 12 to 14 days. Figure 9 shows a growth curve in the productive cultivation.

### EXAMPLES

The present invention now will be further illustrated by, but is by no means limited to, the following Examples. In the Examples, a seed culture for a large-scale production was prepared, and the seed culture was used to produce a large number of mycelia of Tricholoma matsutake.

Tricholoma matsutake FERM BP-7304 used in the Examples was deposited in the International Patent Organism Depositary National Institute of Advanced Industrial Science and Technology [(Former Name) National Institute of Bioscience and Human-Technology Agency of Industrial Science and Technology (Address: AIST Tsukuba Central 6, 1-1, Higashi 1-chome Tukuba-shi, Ibaraki-ken 305-8566 Japan)] on September 14, 2000. Mycological features of Tricholoma matsutake FERM BP-7304 are described in WO02/30440 (patent reference 1). For example, the strain is maintained on 10 mL of an Ebios agar in a glass tube.

Hereinafter Tricholoma matsutake mycelia obtained by cultivation of Tricholoma matsutake FERM BP-7304 and corresponding to the above Matsutake II to VIII are referred to as Matsutake (II-1) to (VIII-1), respectively.

The following fermentors were used in each agitating cultivation step:

In Example 3, Comparative example 2-1, and Comparative example 3-1 (without aeration), a 30-liter jar fermentor equipped with a sparger for aeration and an agitator (6 blade discs; 2 stages) was used in a state in which the sparger for aeration was removed therefrom, and referred to as "30-liter jar fermentor".

In comparative example 3-1, the 30-liter jar fermentor equipped with a sparger for aeration and an agitator (6 blade discs; 2 stages) was used, and referred to as "30-liter jar fermentor with a sparger".

In Example 4, Examples 7-1 to 7-4, Examples 8-2 to 8-3, Examples 9-1 to 9-3, and Example 11-1, a 200-liter fermentor equipped with a sparger for aeration and an agitator (4 blade discs; 2 stages) was used, and referred to as "200-liter fermentor".

In Example 5, a 7-m³ fermentor equipped with a sparger for aeration and an agitator (6 blade discs; 2 stages) was used, and referred to as "7-m³ fermentor".

In Example 6, a 65-m³ fermentor equipped with a sparger for aeration and an agitator (6 blade discs; 3 stages) was used, and referred to as "65-m³ fermentor".

As to the expression "agitation power per unit volume of the culture medium" in Examples 2 to 6, Example 7-1, Example 9-1, Examples 10-1 to 10-3, and Example 11-1, the term "culture medium" in the expression does not mean only a prepared liquid medium, but also the whole contents (including a culture medium of the seed culture) contained in each fermentor, such as 2-liter conical flask, 30-liter jar fermentor, 30-liter jar fermentor with a sparger, 200-liter fermentor, 7-m³ fermentor, or 65-m³ fermentor.

The following substrates for nutrient source used for preparing culture media by using the 200-L fermentor in Example 4, Example 7-1, Example 9-1, or Example 11-1 are referred to as A-type substrates, B-type substrates, or C-type substrates, in necessary.

A-type substrates: (1) potato starch 4.9 kg, (2) glucose 140 g, (3) dried yeast extract 280 g, (4) potassium dihydrogen phosphate 280 g, (5) magnesium sulfate heptahydrate 42 g, (6) calcium chloride dihydrate 0.84 g, (7) zinc sulfate heptahydrate 0.56 g, (8) manganese chloride tetrahydrate 0.70 g, (9) copper sulphate pentahydrate 0.14 g, (10) iron chloride hexahydrate 1.12 g, and (11) thiamin hydrochloride 0.14 g.

B-type substrates: The components and the contents thereof [(1), (2), and (4) to (11)] are the same as the A-type substrates, except for (3) dried yeast extract 320 g.

C-type substrates: The components (4) to (11) and the contents thereof are the same as the A-type substrates and B-type substrates. Further, dried yeast extract 640 g, and potato starch and glucose were contained. Amounts of potato starch and glucose are shown in Examples 11-1 to 11-3.

The procedures in which a production scale was enlarged from a 500-mL conical flask to a 65-m³ fermentor are illustrated in the following Examples 1 to 6.

### Example 1: Process for producing Matsutake (III-1) by stationary liquid cultivation using 500-mL conical flask

Glucose (30 g/L) and dried yeast extract (3 g/L) were dissolved in tap water to prepare a culture medium. An osmotic pressure of the medium after inoculation was 0.4 MPa. The medium was dispensed in 120 mL portions into 500-mL conical flasks, and sterilized. A broth (10 mL) containing Matsutake (II-1) maintained by stationary liquid cultivation was used to inoculate the culture medium. Stationary liquid cultivation was carried out at 23±2°C for 180 days to produce a broth containing Matsutake (III-1) (5 g/L as dried weight).
Propagation rate in inoculation: 13 times.
Concentration of initial mycelia: 0.38 g/L.

### Example 2: Process for producing Matsutake (IV-1) by shaking cultivation

Glucose (30 g/L), dried yeast extract (1 g/L), soybean peptone (2 g/L), malt extract (1 g/L), potassium dihydrogen phosphate (1 g/L), dipotassium hydrogenphosphate (1 g/L), and magnesium sulfate heptahydrate (0.3 g/L) were dissolved in tap water to prepare a culture medium. The medium was dispensed in 870 mL portions into 2-L conical flasks, and sterilized. The broth (130 mL) containing Matsutake (III-1) (5 g/L as dried weight), obtained in Example 1, was homogenized and used to inoculate the culture medium (870 mL). Shaking cultivation (an agitation power per unit volume of the culture medium by a rotary shaker = 0.14 kw/m³) was carried out at 23±2°C for 20 days to produce a broth containing Matsutake (IV-1) (5 g/L as dried weight). In the mycelia, 4.8 g/L of fibrous mycelia were contained. An osmotic pressure of the medium after inoculation was 0.4 MPa.
Propagation rate in inoculation: 7.7 times.
Concentration of initial mycelia: 0.65 g/L.

### Example 3: Process for producing Matsutake (V-1) in 30-L jar fermentor

In a 30-L jar fermentor, potato starch (600 g), glucose (60 g), dried yeast extract (40 g), potassium dihydrogen phosphate (40 g), magnesium sulfate heptahydrate (6 g), calcium chloride dihydrate (0.12 g), zinc sulfate heptahydrate (0.08 g), manganese chloride tetrahydrate (0.1 g), copper sulphate pentahydrate (0.02 g), iron chloride hexahydrate (0.18 g), and thiamin hydrochloride (0.02 g) were dissolved in tap water, and sterilized to prepare a culture medium (18 L). An osmotic pressure of the medium after inoculation was 0.05 MPa.

The broth (2 L) containing Matsutake (IV-1) (5 g/L as dried weight), obtained in Example 2, was used to inoculate the culture medium. Cultivation (agitation power per unit volume of the culture medium = 0.08 kw/m³) was carried out without aeration to the medium from the sparger for aeration at 23±2°C for 7 days to produce a broth containing Matsutake (V-1) (1 g/L as dried weight). In the mycelia, 0.95 g/L of fibrous mycelia were contained.
Propagation rate in inoculation: 10 times.
Concentration of initial mycelia: 0.5 g/L (including 0.45g/L of fibrous mycelia).

### Example 4: Process for producing Matsutake (VI-1) in 200-L fermentor

In a 200-L fermentor, the above A-type substrates were dissolved in tap water, and sterilized to prepare a culture medium (120 L). An osmotic pressure of the medium was 0.05 MPa. The broth (20 L) containing Matsutake (V-1) (1 g/L as dried weight), obtained in Example 3, was used to inoculate the culture medium. Submerged cultivation (an agitation power per unit volume of the culture medium = 0.12 kw/m³) was carried out with aeration at a rate of 42 L/min at 23±2°C for 7 days to produce a broth containing Matsutake (VI-1) (3 g/L as dried weight). In the mycelia, 2.7 g/L of fibrous mycelia were contained.
Propagation rate in inoculation: 7 times.
Concentration of initial mycelia: 0.14 g/L (including 0.13 g/L of fibrous mycelia).

### Example 5: Process for producing Matsutake (VII-1) in 7-m³ fermentor

In a 7-m³ fermentor, soluble starch (140 kg), glucose (4 kg), dried yeast extract (8 kg), potassium dihydrogen phosphate (8 kg), magnesium sulfate heptahydrate (1.2 kg), calcium chloride dihydrate (24 g), zinc sulfate heptahydrate (16 g), manganese chloride tetrahydrate (20 g), copper sulphate pentahydrate (4 g), iron chloride hexahydrate (32 g), and thiamin hydrochloride (4 g) were dissolved in tap water, and sterilized to prepare a culture medium (3.72 m³). An osmotic pressure of the medium was 0.05 MPa. The broth [280 L (corresponding to two tanks)] containing Matsutake (VI-1) (3 g/L as dried weight), obtained in Example 4, was used to inoculate the culture medium. Submerged cultivation (an agitation power per unit volume of the culture medium = 0.05 kw/m³) was carried out with aeration at a rate of at 1.2 m³/min at 23±2°C for 7 days to produce a broth containing Matsutake (VII-1) (3 g/L as dried weight). In the mycelia, 2.6 g/L of fibrous mycelia were contained.
Propagation rate in inoculation: 14.3 times.
Concentration of initial mycelia: 0.21 g/L (including 0.19 g/L of fibrous mycelia).

### Example 6: Process for producing Matsutake (VIII-1) in 65-m³ fermentor

In a 65-m³ fermentor, soluble starch (1575 kg), glucose (45 kg), dried yeast extract (135 kg), potassium dihydrogen phosphate (90 kg), magnesium sulfate heptahydrate (13.5 kg), calcium chloride dihydrate (270 g), zinc sulfate heptahydrate (180 g), manganese chloride tetrahydrate (225 g), copper sulphate pentahydrate (45 g), iron chloride hexahydrate (360 g), and thiamin hydrochloride (45 g) were dissolved in tap water, and sterilized to prepare a culture medium (36 m³). An osmotic pressure of the medium after inoculation was 0.05 MPa.

The broth [4 m³ (corresponding to one tank)] containing Matsutake (VII-1) (3 g/L as dried weight), obtained in Example 5, was used to inoculate the culture medium. Submerged cultivation was carried out with aeration at a rate of at 12 m³/min at 23±2°C for 13 days to produce a broth containing Matsutake (VIII-1) (13.5 g/L as dried weight). The cultivation was started at an agitation power per unit volume of the culture medium of 0.013 kw/m³, and the agitation power was increased, in accordance with the growth of mycelia, to 0.12 kw/m³ on the 12th day of the cultivation.
Propagation rate in inoculation: 10 times.
Concentration of initial mycelia: 0.3 g/L (including 0.26 g/L of fibrous mycelia).

### Comparative example 1

### Effects of the broth containing Matsutake (III-1) produced by stationary liquid cultivation on shaking cultivation: Comparison with a broth not produced via the stationary liquid cultivation step

The shaking cultivation for 20 days described in Example 2 was repeated except that an Ebios agar (10 mL) containing Matsutake (II-1) was used.

However, the amount of dried mycelia contained in the broth obtained after the cultivation for 20 days could not be determined.

### Example 7

### Effects of a production period of the broth containing Matsutake (III-1) produced by stationary liquid cultivation on submerged cultivation in the 200-L fermentor: Comparisons between the stationary liquid cultivation for 180 days in Example 1, and stationary liquid cultivation for 30, 50, or 400 days

### Example 7-1: Stationary liquid cultivation for 180 days

The procedures described in Examples 1 to 3 were repeated to produce a broth containing Matsutake (V-1).

In a 200-L fermentor, the above B-type substrates were dissolved in tap water, and sterilized to prepare a culture medium (120 L). The broth (20 L) containing Matsutake (V-1) was used to inoculate the culture medium. Submerged cultivation (an agitation power per unit volume of the culture medium = 0.12 kw/m³) was carried out with aeration at a rate of 42 L/min at 23±2°C for 12 days to produce a broth containing mycelia (12 g/L as dried weight).
Propagation rate in inoculation: 7 times.
Concentration of initial mycelia: 0.14 g/L (including 0.13 g/L of fibrous mycelia).

### Example 7-2: Stationary liquid cultivation for 30 days

The procedure described in Example 1 was repeated except that a period of the stationary liquid cultivation was 30 days. Further, the procedures described in Examples 2 and 3 were repeated to produce a broth. The cultivation in the 200-L fermentor described in Example 7-1 was repeated except that the broth (20 L) was used to produce a broth containing mycelia (2 g/L as dried weight).

### Example 7-3: Stationary liquid cultivation for 50 days

The procedure described in Example 1 was repeated except that a period of the stationary liquid cultivation was 50 days. Further, the procedures described in Examples 2 and 3 were repeated to produce a broth. The cultivation in the 200-L fermentor described in Example 7-1 was repeated except that the broth (20 L) was used to produce a broth containing mycelia (8 g/L as dried weight).

### Example 7-4: Stationary liquid cultivation for 400 days

The procedure described in Example 1 was repeated except that a period of the stationary liquid cultivation was 400 days. Further, the procedures described in Examples 2 and 3 were repeated to produce a broth. The cultivation in the 200-L fermentor described in Example 7-1 was repeated except that the broth (20 L) was used to produce a broth containing mycelia (6 g/L as dried weight).

### Comparative example 2

### Effects of the broth containing Matsutake (IV-1) produced by shaking cultivation on submerged cultivation: Comparison with a broth not produced via the shaking cultivation step

### Comparative example 2-1: Not via the shaking cultivation step

The broth (2 L) containing Matsutake (III-1) (5 g/L as dried weight), obtained in Example 1, was homogenized. The homogenized broth was used to carry out cultivation in a 30-L jar fermentor in accordance with the procedure described in Example 3. However, the amount of dried mycelia contained in the broth obtained after the cultivation for 7 days could not be determined.

In this connection, the results obtained via the shaking cultivation for 20 days are shown in Example 7-1. The data in inoculation and after cultivation were as follows:
A broth containing mycelia (12 g/L as dried weight) was obtained.
Propagation rate in inoculation: 7 times.
Concentration of initial mycelia: 0.14 g/L (including 0.13 g/L of fibrous mycelia).

### Example 8

### Effects of the broth containing Matsutake (IV-1) produced by shaking cultivation on submerged cultivation in the 200-L fermentor: Comparisons between the shaking cultivation for 20 days in Example 2, and shaking cultivation for 5 or 3 days

### Example 8-1: Shaking cultivation for 20 days

The results are shown in Example 7-1. The data in inoculation and after cultivation were as follows:
A broth containing mycelia (12 g/L as dried weight) was obtained.
Propagation rate in inoculation: 7 times.
Concentration of initial mycelia: 0.14 g/L (including 0.13 g/L of fibrous mycelia).

### Example 8-2: Shaking cultivation for 5 days

The procedure described in Example 2 was repeated using the broth produced in Example 1, except that a period of the shaking cultivation was 5 days. Further, the procedure described in Example 3 was repeated to produce a broth. The broth was used to carry out cultivation in a 200-L fermentor in accordance with the procedure described in Example 7-1 to produce a broth containing mycelia (7 g/L as dried weight).

### Example 8-3: Shaking cultivation for 3 days

The procedure described in Example 2 was repeated using the broth produced in Example 1, except that a period of the shaking cultivation was 3 days. Further, the procedure described in Example 3 was repeated to produce a broth. The broth was used to carry out cultivation in a 200-L fermentor in accordance with the procedure described in Example 7-1 to produce a broth containing mycelia (0.5 g/L as dried weight).

### Comparison Example 3

### Effects of the broth containing Matsutake (V-1) produced by agitating cultivation without aeration in the 30-L jar fermentor, on submerged cultivation in the 200-L fermentor: Comparison with a broth produced with aeration in a 30-liter jar fermentor with a sparger

### Comparison example 3-1: Cultivation of Matsutake (IV-1) with aeration

The procedure described in Example 3 was repeated using the broth produced in accordance with the procedures described in Examples 1 and 2, except that the 30-liter jar fermentor with a sparger was used and that aeration at a rate of 2 L/min was carried out, to produce a broth.
Mycelia in the broth flocculated. The amount of dried mycelia after the cultivation was 0.6 g/L (including 0.03 g/L of fibrous mycelia). Further, the broth was used to carry out cultivation in a 200-L fermentor in accordance with the procedure described in Example 7-1 to produce a broth containing mycelia (5 g/L as dried weight).

In this connection, the results obtained without aeration in the cultivation of Matsutake (IV-1) are shown in Example 7-1. As with Example 3, most mycelia in the broth produced in the 30-liter jar fermentor without aeration were fibrous. The amount of dried mycelia after the cultivation was 1 g/L (including 0.95 g/L of fibrous mycelia). The data in inoculation and after cultivation were as follows:
A broth containing Mycelia (12 g/L as dried weight) was obtained.
Propagation rate in inoculation: 7 times.
Concentration of initial mycelia: 0.14 g/L (including 0.13 g/L of fibrous mycelia).

### Example 9

### Effects of a volume of the broth containing Matsutake (VI-1) produced by submerged cultivation with aeration on submerged cultivation in the 200-L fermentor: Comparisons between various volumes of broths used in Example 4

### Example 9-1: Inoculation with 10 L of broth containing Matsutake (VI-1)

The procedures described in Examples 1 to 4 were repeated to produce a broth containing Matsutake (VI-1).

In a 200-L fermentor, the B-type substrates were dissolved in tap water, and sterilized to prepare a culture medium (130 L). The broth (10 L) containing Matsutake (VI-1) was used to inoculate the culture medium (130 L). Submerged cultivation (an agitation power per unit volume of the culture medium = 0.12 kw/m³) was carried out with aeration at a rate of 42 L/min at 23±2°C for 12 days to produce a broth containing mycelia (12 g/L as dried weight).
Propagation rate in inoculation: 14 times.
Concentration of initial mycelia: 0.21 g/L (including 0.19 g/L of fibrous mycelia).

### Example 9-2: Inoculation with 5 L of broth containing Matsutake (VI-1)

The cultivation in the 200-L fermentor was carried out in accordance with the procedure described in Example 9-1, except that the broth (5 L) containing Matsutake (VI-1) produced by the procedures described in Examples 1 to 4 was used together with sterile water (5 L), to produce a broth containing mycelia (10 g/L as dried weight).
Propagation rate in inoculation: 28 times.
Concentration of initial mycelia: 0.107 g/L (including 0.097 g/L of fibrous mycelia).

### Example 9-3: Inoculation with 2 L of broth containing Matsutake (VI-1)

The cultivation in the 200-L fermentor was carried out in accordance with the procedure described in Example 9-1, except that the broth (2 L) containing Matsutake (VI-1) produced by the procedures described in Examples 1 to 4 was used together with sterile water (8 L), to produce a broth containing mycelia (2 g/L as dried weight).
Propagation rate in inoculation: 70 times.
Concentration of initial mycelia: 0.043 g/L (including 0.04 g/L of fibrous mycelia).

### Example 10

### Effects of agitating conditions in submerged cultivation by using, as a seed culture, the broth containing Matsutake (VI-1) produced by submerged cultivation with aeration, with respect to mycelia growth in the 200-L fermentor:

### Example 10-1: Agitation power per unit volume of a culture medium of 0.12 kW/m³

The procedures described in Example 9-1 were repeated. The data in inoculation and after cultivation were as follows:
A broth containing mycelia (12 g/L as dried weight) was obtained.
Propagation rate in inoculation: 14 times.
Concentration of initial mycelia: 0.21 g/L (including 0.19 g/L of fibrous mycelia).

### Example 10-2: Agitation power per unit volume of a culture medium of 1.09 kW/m³

The procedures described in Example 10-1 were repeated, except that the agitation power per unit volume of a culture medium was 1.09 kW/m³ when carrying out submerged cultivation for 12 days. A broth containing mycelia (7 g/L as dried weight) was obtained.
Propagation rate in inoculation: 14 times.
Concentration of initial mycelia: 0.21 g/L (including 0.19 g/L of fibrous mycelia).

### Example 10-3: Agitation power per unit volume of a culture medium of 2.63 kW/m³

The procedures described in Example 10-1 were repeated, except that the agitation power per unit volume of a culture medium was 2.63 kW/m³ when carrying out submerged cultivation for 12 days. A broth containing mycelia (7 g/L as dried weight) was obtained. However, the amount of dried mycelia contained in the broth obtained after the cultivation for 12 days could not be determined.

### Example 11

### Effects of various carbon sources in submerged cultivation by using, as a seed culture, the broth containing Matsutake (VI-1) produced by submerged cultivation with aeration, with respect to mycelia growth in the 200-L fermentor: Comparison between the amount of potato starch and that of glucose (as an osmotic pressure)

### Example 11-1: Osmotic pressure of 0.05 MPa

The procedures described in Examples 1 to 4 were repeated to produce a broth containing Matsutake (VI-1).

In a 200-L fermentor, the C-type substrates, 9.8 kg of potato starch, and 140 g of glucose were dissolved in tap water, and sterilized to prepare a culture medium (130 L) having an osmotic pressure of 0.05 MPa. The broth (10 L) containing Matsutake (VI-1) was used to inoculate the culture medium (130 L). Submerged cultivation (an agitation power per unit volume of the culture medium = 0.12 kw/m³) was carried out with aeration at a rate of 42 L/min at 23±2°C for 12 days to produce a broth containing mycelia (14 g/L as dried weight).
Propagation rate in inoculation: 14 times.
Concentration of initial mycelia: 0.21 g/L (including 0.19 g/L of fibrous mycelia).

### Example 11-2: Osmotic pressure of 0.50 MPa

The procedures described in Example 11-1 were repeated, except that 4.97 kg of potato starch and 4.97 kg of glucose were used to prepare a culture medium (130 L) having an osmotic pressure of 0.50 MPa, when carrying out submerged cultivation for 12 days. A broth containing mycelia (9.5 g/L as dried weight) was obtained.
Propagation rate in inoculation: 14 times.
Concentration of initial mycelia: 0.21 g/L (including 0.19 g/L of fibrous mycelia).

### Example 11-3: Osmotic pressure of 0.98 MPa

The procedures described in Example 11-1 were repeated, except that 140 g of potato starch and 9.8 kg of glucose were used to prepare a culture medium (130 L) having an osmotic pressure of 0.98 MPa, when carrying out submerged cultivation for 12 days. A broth containing mycelia (2 g/L as dried weight) was obtained.
Propagation rate in inoculation: 14 times.
Concentration of initial mycelia: 0.21 g/L (including 0.19 g/L of fibrous mycelia).

### Example 12

### Effects of agitation power in agitating cultivation on form and growth activity of Matsutake (V):

### Example 12-1: Agitation power of 1.6 kw/m³

The procedures described in Example 2 were repeated, except that the agitation power per unit volume of a culture medium in the shaking cultivation was 1.6 kw/m³, to produce a broth containing Matsutake (IV-1) (3.2 g/L as dried weight) including 2.2 g/L of fibrous mycelia. Then the procedures described in Example 3 were repeated, except that 3 L (corresponding to the concentration of initial mycelia in Example 3) of the broth containing Matsutake (IV-1) were used for inoculation, to produce a broth containing Matsutake (V-1) in the 30-L jar fermentor. In the broth, mycelia (0.75 g/L as dried weight) including 0.6 g/L of fibrous mycelia were contained.
Propagation rate in inoculation: 6.7 times.
Concentration of initial mycelia: 0.48 g/L (including 0.38 g/L of fibrous mycelia).

The Matsutake (V) was used to repeat the procedures described in Example 7-1, except that 20 L of the Matsutake (V) was used for inoculation in the 200-L fermentor. As a result, a broth containing mycelia (9.7 g/L as dried weight) was obtained.

### Example 12-2: Agitation power of 2.2 kw/m³

The procedures described in Example 2 were repeated, except that the agitation power per unit volume of a culture medium in the shaking cultivation was 2.2 kw/m³ (i.e., a high speed agitation), to produce a broth containing Matsutake (IV-1) (2.4 g/L as dried weight) including 0.013 g/L of fibrous mycelia. Then the procedures described in Example 3 were repeated, except that 4 L (corresponding to the concentration of initial mycelia in Example 3) of the broth containing Matsutake (IV-1) were used for inoculation, to produce a broth containing Matsutake (V-1) in the 30-L jar fermentor. In the broth, mycelia (0.5 g/L as dried weight) including 0.01 g/L of fibrous mycelia were contained.
Propagation rate in inoculation: 5 times.
Concentration of initial mycelia: 0.48 g/L (including 0.0026 g/L of fibrous mycelia).

The Matsutake (V) was used to repeat the procedures described in Example 7-1, except that 20 L of the Matsutake (V) was used for inoculation in the 200-L fermentor. As a result, a broth containing mycelia (4.7 g/L as dried weight) was obtained.

### Example 13

### Effects of agitation power for Matsutake (VI) in 200-L fermentor on form and growth activity of Matsutake (VI):

### Example 13-1: Agitation power of 1.7 kw/m³

The procedures described in Examples 1 to 3 were repeated to produce a broth containing Matsutake (V-1). The procedures described in Example 4 were repeated, except that the agitation power per unit volume of a culture medium in the shaking cultivation was 1.7 kw/m³ in the 200-L fermentor, to produce a broth containing Matsutake (VI-1) (1.6 g/L as dried weight) including 1.1 g/L of fibrous mycelia. The Matsutake (VI-1) was used to repeat the procedures described in Example 9-1 to obtain a broth containing mycelia (9.7 g/L as dried weight).
Propagation rate in inoculation: 14 times.
Concentration of initial mycelia: 0.11 g/L (including 0.78 g/L of fibrous mycelia).

### Example 13-2: Agitation power of 2.2 kw/m³

The procedures described in Examples 1 to 3 were repeated to produce a broth containing Matsutake (V-1). The procedures described in Example 4 were repeated, except that the agitation power per unit volume of a culture medium in the shaking cultivation was 2.2 kw/m³ in the 200-L fermentor, to produce a broth containing Matsutake (VI-1) (0.68 g/L as dried weight) including 0.02 g/L of fibrous mycelia. The procedures described in Example 9-1 were repeated, except that 25 L of the obtained Matsutake (VI-1) was used for inoculation, and that 115 L of medium was used to obtain a broth containing mycelia (2.5 g/L as dried weight).
Propagation rate in inoculation: 5.6 times.
Concentration of initial mycelia: 0.12 g/L (including 0.0036 g/L of fibrous mycelia).

### Example 14: Evaluation of Tricholoma matsutake mycelia obtained in mass cultivation for natural killer (NK) cell activity

### (1) Preparation of lyophilized Tricholoma matsutake mycelia obtained by mass cultivation

The Tricholoma matsutake mycelia obtained in Example 6 was centrifuged by a basket centrifuge), and the collected cake was crushed into small blocks having a length of approximately 5 mm. A tray (510 mm × 790 mm) for lyophilization was filled with 6 kg of the crushed blocks of Tricholoma matsutake mycelia [water content = 79.4% wet base (W.B.)], and prefrozen at -35°C for 24 hours. The prefrozen mycelia were lyophilized under a reduced pressure of 13 Pa to obtain 1.2 kg of lyophilized mycelia. In the lyophilization, the maximum temperature was 50°C and the time required for the lyophilization was 24 hours. The water content of the lyophilized mycelia was 2.7% W.B.

The resulting lyophilized mycelia (1.2 kg) was crushed by a crusher (pin mill; 5000 r/min) to obtain powder. In the crushing treatment, the lyophilized mycelia were supplied at a rate of 12 kg/h, and the temperature was controlled at 50°C or less. The particle size of the powder was such that 90% thereof passed through a mesh filter having a mesh opening of 125 µm.

### (2) Evaluation for NK cell activity

In this Example, an NK cell activity was evaluated as a physiological activity of Tricholoma matsutake mycelia. The NK cell activity is known as an index of an activity of promoting a recovery from stress. The NK cell activity was measured in accordance with a method (an evaluation system using mice) described in Evaluation Example 1 of WO02/30440. More particularly, "Lytic Units 30% (LU30)", that is, the number of cells which kill 30% tumor cells per 10⁷ cells of effector cells, was calculated, and the obtained values were compared by t-test. In the experiment, a dose of Tricholoma matsutake mycelia power was 150 mg/kg/day per mouse (corresponding to 0.6 g/day in human).

As a result, lyophilized powder of Tricholoma matsutake mycelia prepared in Example 14(1) exhibited the NK cell activity, and a significant difference (P < 0.05) with respect to a control group in which distilled water was administered was confirmed.

### INDUSTRIAL APPLICABILITY

According to the process of the present invention, a large number of Matsutake mycelia can be produced without a loss of physiological activities. For example, Matsutake mycelia as a seed culture can be produced by cultivating mycelia cultivated or maintained in a solid or liquid medium, in a liquid medium under stationary conditions, and cultivating the obtained mycelia under shaking, and further cultivating the mycelia on a small scale (for example, by using a small-sized fermentor having a capacity of less than 100 L) under agitation without aeration or with aeration at a low rate of less than 0.05 vvm in a liquid medium. Further, the obtained seed culture can be cultivated under submerged conditions on a large scale (for example, by using a middle-sized or large-sized fermentor) to produce a large number of Matsutake mycelia.

Although the present invention has been described with reference to specific embodiments, various changes and modifications obvious to those skilled in the art are possible without departing from the scope of the appended claims.

## Claims

1. A process for producing a mycelium of a Matsutake fungus, comprising the step of cultivating a mycelium on a small scale under agitation without aeration in a liquid medium or with aeration at a low rate of less than 0.05 vvm.

2. The process according to claim 1, further comprising, as a precultivation step before the agitating cultivation step,
(1) cultivating a mycelium in a liquid medium under stationary conditions,
(2) cultivating a mycelium under shaking, or
(3) cultivating a mycelium in a liquid medium under stationary conditions, and further cultivating the obtained mycelium under shaking.

3. The process according to claim 1 or 2, wherein an agitation power per unit volume of a culture medium in the agitating cultivation step is 0.01 to 2 kW/m³.

4. A process for producing a mycelium of a Matsutake fungus, comprising the steps of:
cultivating a mycelium in a liquid medium under stationary conditions, and
cultivating the obtained mycelium under shaking.

5. A process for producing a mycelium of a Matsutake fungus, comprising the step of cultivating the mycelium obtained by the process according to any one of claims 1 to 4 as a seed culture under submerged conditions.

6. The process according to any one of claims 2 to 5, wherein a period of the stationary cultivation step is 30 to 400 days.

7. The process according to any one of claims 2 to 6, wherein a period of the shaking cultivation step is 5 to 50 days.

8. The process according to any one of claims 1 to 7, wherein a propagation rate in inoculation is 2 to 50 times.

9. The process according to any one of claim 1 to 8, wherein an osmotic pressure of a culture medium is 0.01 to 0.8 MPa.

10. The process according to any one of claim 1 to 9, wherein a concentration of a fibrous mycelium contained in an initial mycelium is 0.05 g/L or more.
